# EUROPEAN PATENT APPLICATION

(11) **EP 2 857 022 A1**
(43) Date of publication of application: **08.04.2015**
(21) Application number: 14196879.2
(22) Date of filing: 14.05.2010
(51) Int. Cl.: A61K 31/7004, A61K 33/24, A61P 5/00

(54) **L-arabinose plus chromium for controlling the metabolization of sucrose**

(30) Priority: 14.05.2009 US 178268 P
(62) Divisional of application: 10775598.5
(71) Applicant: Pharmachem Laboratories, Inc., Kearny, NJ 07032 (US)
(72) Inventor: Skop, Mitch, Closter, NJ New Jersey 07624 (US)
(74) Representative: Jansen, Cornelis Marinus

(57) **Abstract**

A composition is provided for use in decreasing metabolization of sucrose in a diabetic or obese host. The composition comprises, as the active ingredients, 500 mg to 1000 mg L-Arabinose and 100 µg to 200 µg chromium. The composition is also provided for use in inhibiting glycemic response to a meal containing sucrose in a diabetic or obese host.

## Description

### CROSS-REFERENCE TO RELATED APPLICATION

This application claims priority benefit of U.S. Provisional Application No. 61/178,268, filed May 14, 2009.

### BACKGROUND OF THE INVENTION

The glycemic index, or GI, is a measure of the effects of carbohydrates on blood glucose levels. Carbohydrates that break down rapidly during digestion, release glucose rapidly into the bloodstream, and have a high GI. Carbohydrates that break down slowly, release glucose gradually into the bloodstream, and have a low GI. Sucrose, or table sugar, has the highest GI amongst carbohydrates.

Sucrose is a compound molecule that consists of one glucose molecule bound to a fructose molecule.

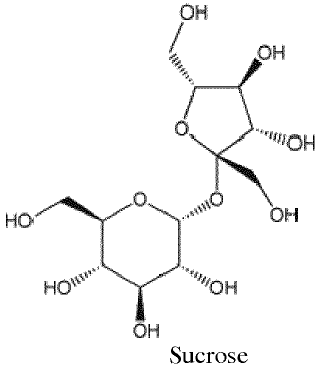

Upon ingesting sucrose, digestive enzymes and acids in the body break the sucrose down into its components of glucose and fructose. Free glucose molecules in the body signal many events as will be described below.

Several lines of recent scientific evidence have shown that individuals who follow a low GI diet over many years are at a significantly lower risk for developing both type 2 diabetes and coronary heart disease than those who follow a higher GI diet. High blood glucose levels or repeated glycemic "spikes" following a meal may promote these diseases by increasing oxidative damage to the vasculature and also by a direct increase in insulin levels.

Recent animal research provides compelling evidence that a high GI diet is associated with an increased risk of obesity. When the body is provided with excess amounts of high GI foods, such as simple carbohydrates and foods containing sugar (sucrose) which are quickly reduced to glucose, there is no need for the body to utilize stored fats and/or protein to derive energy.

Excess glucose in the bloodstream signals the body to produce insulin. This rise in insulin encourages hunger, increases the body's tendency to convert calories into body fat, and reduces the body's ability to burn calories. Of particular importance, it has been shown that excessive amounts of circulating insulin appears to be a contributor to insulin resistance via down-regulation of insulin receptors. Poor glucose control and insulin resistance are two of the most striking biomarkers and end points associated with chronic diseases.

Research suggests following a low carbohydrate/low sugar diet to avoid the detrimental effects of glycemic spikes. It has been suggested that certain nutritional supplements may be beneficial at controlling the glycemic response to simple carbohydrates and/or sugars. For example, some studies have reported that different forms of chromium may provide improved glucose control in humans. However, it is thought that the beneficial effects of chromium may only be seen in persons with impaired glucose tolerance, e.g. diabetes.

Recent studies have also reported that L-Arabinose may suppress serum glucose levels after ingesting sucrose.

Presently, there is a need for new and improved dietary supplements that can decrease the rate at which sucrose is metabolized into glucose.

### SUMMARY OF THE INVENTION

It is an object of the present invention to provide a composition comprising chromium and L-Arabinose.

It is also an object of the invention to provide a method for decreasing the metabolization of sucrose in a host in need thereof by administering a composition comprising chromium and L-Arabinose to the host.

A further object of the invention is to provide a method for inhibiting the glycemic response to a meal containing sucrose in a host in need thereof by administering a composition comprising chromium and L-Arabinose to the host.

### DETAILED DESCRIPTION OF THE INVENTION

It has now been discovered that the metabolization of sucrose can be decreased in a host in need thereof by administering an effective amount of a composition comprising L-Arabinose and chromium to said host.

### L-Arabinose

According to the invention, L-Arabinose is a crystalline pentose sugar having the formula C₅H₁₀O₅, and the following structure:

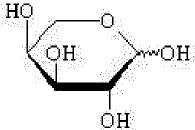

L-Arabinose is a non-caloric sweetener which has taste characteristics similar to sucrose and shows little absorbability. L-Arabinose inhibits enzymes which hydrolyze dissacharides such as sucrose.

The L-Arabinose of the instant invention can be derived from any known source including, for example, arabinan, arabinoxylan, arabinogalactan and hemicellulose of higher plants. L-Arabinose can also be obtained in a free state from fermented foods such as miso and sake.

### Chromium

According to the invention, chromium can be in any ingestible, known form, for example, chromium picolinate, chromium chloride, chromium niacin amino acid chelate and/or chromium nicotinate.

Chromium is a mineral that humans require in trace amounts, although its mechanisms of action in the body, and the amounts needed for optimal health are not well defined.

Chromium is known to enhance the action of insulin, a hormone critical to the metabolism and storage of carbohydrate, fat, and protein in the body.

Chromium is widely distributed in the food supply, but most foods provide only small amounts (less than 2 micrograms per serving). Meat and whole-grain products, as well as some fruits, vegetables, and spices are relatively good sources. In contrast, foods high in simple sugars (like sucrose and fructose) are low in chromium.

### Sucrose

According to the invention, sucrose (common name: table sugar, also called saccharose) is a disaccharide of glucose and fructose, with the molecular formula C₁₂H₂₂O₁₁. In mammals, sucrose is very readily digested in the stomach into its component sugars, by acidic hydrolysis. This step is performed by a glycoside hydrolase, which catalyzes the hydrolysis of sucrose to the monosaccharides glucose and fructose. This is also known as metabolization of sucrose.

Glucose and fructose are rapidly absorbed into the bloodstream in the small intestine. Undigested sucrose passing into the intestine is also broken down by sucrase or isomaltase glycoside hydrolases, which are located in the membrane of the microvilli lining the duodenum. These products are also transferred rapidly into the bloodstream.

### Synergy

Applicants have discovered that the combination of L-Arabinose and chromium in a composition provides a synergistic effect on decreasing the metabolization of sucrose in a host. Applicants have also discovered that a composition containing L-Arabinose and chromium synergistically inhibits the glycemic response in a host to a meal containing sucrose.

The synergistic effect of the combination of L-Arabinose and chromium is greater than the effect of either L-Arabinose or chromium alone.

### Glycemic Response

According to the invention, glycemic response is the biological response that occurs in a host after a certain food is consumed. The glycemic response of a certain food can be measured by the time it takes to convert that food into glucose. Accordingly, the glycemic response of a food is a measure of the food's ability to elevate blood sugar.

As discussed above, high glycemic foods enter the bloodstream quickly, and increase blood sugar levels rapidly. Low glycemic foods enter the bloodstream slowly, and help maintain stable blood sugar levels. Sucrose is amongst the highest glycemic foods known.

### Host

According to the invention, a method for decreasing metabolization of sucrose in a host in need is provided. A host in need can be any mammal that could benefit from decreasing the breakdown of sucrose into glucose as a result of ingesting a food containing sucrose. For example, a host in need thereof can be a diabetic or an obese individual. The host in need can also be any individual that desires a decrease in the breakdown of sucrose into glucose as a result of ingesting a food containing sucrose, for any other health concern, or to optimize their health.

According to the invention, a method for inhibiting the glycemic response to a meal containing sucrose, in a host in need thereof, is provided. A host in need can be any mammal that could benefit from inhibiting the glycemic response to a meal containing sucrose. For example, a host in need thereof can be a diabetic or an obese individual. The host in need can also be any individual that desires inhibition of the glycemic response to a meal containing sucrose, for any other health concern, or to optimize their health.

A host can be any mammal. Mammals include, for example, humans, as well as pet animals such as dogs and cats, laboratory animals such as rats and mice, and farm animals such as horses and cows. Humans are most preferred.

### Effective Amounts

The composition of the invention comprises an admixture of L-Arabinose and chromium, where both ingredients are present in effective amounts. Optimal doses of the chromium can be determined by one skilled in the art based on a number of parameters including, for example, age, sex, weight, condition being treated, the severity of the condition, and the route of administration. For example the amount of chromium present in the composition is from about 100 µg to about 200 µg

Optimal doses of L-Arabinose can be determined by one skilled in the art based on a number of parameters including, for example, age, sex, weight, condition being treated, the severity of the condition, and the route of administration. For example the amount of L-Arabinose present in the composition is from about 500 mg to about 1000 mg.

According to the invention, an effective amount of chromium and L-Arabinose is any amount that exhibits synergistic effects on inhibiting the metabolization of sucrose, and/or inhibiting the glycemic response to a meal containing sucrose.

For example, a preferred effective amount of chromium is about 100µg, and a preferred effective amount of L-Arabinose is about 1000mg.

### Administration

The composition of the invention can be administered topically or systemically. Systemic administration can be enteral or parenteral. Enteral administration is preferred. For example, the composition can be easily administered orally. Liquid or solid (e.g., tablets, gelatin capsules) formulations can be employed. The formulation can include pharmaceutically acceptable excipients, adjuvants, diluents, or carriers. The composition can also be administered intravenously, with a suitable pharmaceutical carrier (vehicle) or excipient, as understood by those skilled in the art. Topical administration can be, for example, in a cream or emollient.

Administration of the composition can take place before, during or after a meal. In a preferred embodiment, the composition is administered prior to a meal. For example, the composition is administered about one hour prior to a meal. More preferably, the composition is administered about 30 minutes prior to a meal. The composition can also be administered about 15 minutes prior to a meal. In another embodiment, the composition is administered during a meal.

### EXAMPLES

### Example 1

A study was conducted of 50 healthy, non-diabetic adult subjects. The subjects included 30 women and 20 men between ages 18 and 70.

Each subject completed a quality of life survey. Then, a DXA (Dual-energy X-ray Absorptiometry) Body Composition test measuring the total and regional body fat, lean mass, and bone density was performed on each subject.

After fasting for 12 hours, a venous insulin test was performed as well as a capillary "finger-stick" blood glucose test. Immediately following the tests, subjects consumed 70 grams of sugar in 150 grams of bottled water (70g sucrose challenge) (t=0). At 30 and 60 minutes following consumption, additional venous insulin tests were performed. Additional capillary blood glucose tests were preformed at 30, 45, 60, and 90 minutes following consumption.

On day two, the same tests were performed, except at t=0 the subjects consumed the 70 gram sugar solution which also contained an L-Arabinose and chromium supplement (LA-Cr). The L-Arabinose and chromium supplement (LA-Cr) consisted of 1.0 g L-Arabinose and 200 µg chromium.

Consumption of LA-Cr simultaneously in the sugar solution suppressed the glucose response in all four test time periods compared to the control values. Table 1, below, shows the percentage reduction at each time, the average percentage reduction, and the percentage reduction in the area under the curve (AUC) using two methods of estimating AUC from the data.

**Table 1: Effects of LA-Cr on changes from baseline in capillary glucose levels when consumed with a 70g sucrose challenge.**

| Ref | Measurement Times (min after t=0 | 30 | 45 | 60 | 90 | Average | AUC-1 | AUC-2 |
|---|---|---|---|---|---|---|---|---|
| 1 | Control | 53.1 | 54.2 | 44.6 | 23.2 | | 3338 | 170.8 |
| 2 | Treatment (LA-Cr), n=50 | 42.9 | 40.0 | 33.6 | 16.9 | | 2726 | 128.8 |
| 3 | Difference | -10.1 | -14.1 | -11.0 | -6.3 | | -612 | -42.1 |
| 4 | % Difference | -19.1% | -26.1% | -24.8% | -27.1% | -20.0% | -18.4% | -24.6% |
| 5 | Significance (*P*) | *P* <0.01 | *P* <0.001 | *P* <0.01 | *P* <0.05 | *P* <0.001 | *P* <0.01 | *P* <0.0001 |

The study of insulin changes from baseline also showed suppression. The results for suppression after 60 minutes and for suppression of area under the curve were statistically significant. The results after 30 minutes showed suppression, but the result was not statistically significant (*P*=0.246). The results are show below in Table 2.

**Table 2: Effects of LA-Cr on changes from baseline in serum insulin when consumed with a 70g sucrose challenge**

| Ref | Measurement Times (min after t=0) | 30 | 60 | Average | AUC-1 | AUC-2 |
|---|---|---|---|---|---|---|
| 1 | Treatment (LA-Cr), n=49 | -11.9% | -28.2% | -20.0% | -28.2% | -20.0% |
| 2 | Significance *P* (NS=not significant) | NS | *P*<0.001 | *P*<0.01 | *P*<0.001 | *P*<0.01 |

Consumption of the LA-Cr supplement in conjunction with 70grams of sugar leads to a significant reduction of the glucose and insulin response to the sugar challenge.

### Example 2

A study of 20 subjects (14 women, 6 men, between ages 36 and 67) was conducted over a 28-day period. As in the study above, the control measurements were taken on day one with the administration of the 70g sugar solution and for each subsequent day the subjects were administered LA-Cr in the sugar solution and measurements were taken as described above in example 1.

On the first and last days, the following tests were performed on all subjects: 44-blood chemistry panel and DXA test. On both days, each subject filled out a Quality of Life Questionnaire.

The results indicated that consuming the LA-Cr supplement over a 4 week period did not cause any adverse effect on blood chemistries, body composition, or self-report quality of life.

### Features of the Parent Application

1. A composition comprising L-Arabinose and chromium.
2. The composition according to feature 1, wherein the L-Arabinose is in an amount of about 1000 mg.
3. The composition according to feature 1, wherein the chromium is in an amount of about 100 mg.
4. The composition according to feature 1, wherein the chromium is selection from the group consisting of chromium picolinate, chromium chloride, chromium niacin amino acid chelate and chromium nicotinate.
5. A method for decreasing metabolization of sucrose in a host in need thereof comprising administering an effective amount of a composition comprising L-Arabinose and chromium to said host.
6. The method according to feature 5, wherein the L-Arabinose is in an amount of about 1000 mg.
7. The method according to feature 5, wherein the chromium is in an amount of about 100 µg.
8. The method according to feature 5, wherein the administration occurs before a meal.
9. A method for inhibiting glycemic response to a meal containing sucrose in a host in need thereof comprising administering an effective amount of a composition comprising L-Arabinose and chromium to said host.
10. The method according to feature 9, wherein the L-Arabinose is in an amount of about 1000 mg.
11. The method according to feature 9, wherein the chromium is in an amount of about 100 µg.
12. The method according to feature 9, wherein the administration occurs before a meal.

## Claims

1. A composition comprising, as the active ingredients, 500 mg to 1000 mg L-Arabinose and 100 µg to 200 µg chromium for use in decreasing metabolization of sucrose in a diabetic or obese host.

2. A composition comprising, as the active ingredients, 500 mg to 1000 mg L-Arabinose and 100 µg to 200 µg chromium for use in inhibiting glycemic response to a meal containing sucrose in a diabetic or obese host.

3. The composition for use according to claim 1 or claim 2, wherein the L-Arabinose is in an amount of about 1000 mg.

4. The composition for use according to claim 1 or claim 2, wherein the chromium is in an amount of about 100 µg.

5. The composition for use according to claim 1 or claim 2, wherein the composition is to be administered to said host before a meal.

6. The composition for use according to any one of the preceding claims, wherein the chromium is selected from the group consisting of chromium picolinate, chromium chloride, chromium niacin amino acid chelate and chromium nicotinate.
